# EUROPEAN PATENT APPLICATION

(11) **EP 3 243 469 A1**
(43) Date of publication of application: **15.11.2017**
(21) Application number: 15876418.3
(22) Date of filing: 15.02.2015
(51) Int. Cl.: A61B 18/12, A61B 17/94

(54) **MUCOSA DISSECTOR**

(30) Priority: 05.01.2015 CN 201510004876
(71) Applicant: Jiangsu Vedkang Medical Science & Technology Co., Ltd, Changzhou, Jiangsu 213100 (CN); Zhang, Jianguo, Beijing 100039 (CN)
(72) Inventor: SHAO, Ke, Changzhou Jiangsu 213100 (CN)
(74) Representative: dompatent von Kreisler Selting Werner - Partnerschaft von Patent- und Rechtsanwälten mbB
(86) International application number: PCT/CN2015/000098
(87) International publication number: WO 2016/109907

(57) **Abstract**

A mucosa dissector comprises an outer sleeve assembly, an electrical loop (2), a sliding ring (8) and a pulling cable (4). At a portion of the electrical loop (2) is provided an insulated layer (1), and the rest of the electrical loop is exposed so as to dissect the mucosa. The sliding ring (8) is provided on the outer sleeve assembly, and is slidable axially relative to the outer sleeve assembly; an electrode plug (7) is provided on the sliding ring (8). The pulling cable (4) is movable axially to be inserted into the outer sleeve assembly. One end of the pulling cable (4) is connected with the electrical loop (2), and the other end is electrically connected with the electrode plug (7) on the sliding ring (8). The mucosa dissector can contact the tissue easily and be positioned accurately when used to dissect the submucosa tissue, which facilitates the operation, and avoids the phenomenon of burning out the healthy tissues.

## Description

### Technical field

The invention relates to a mucosa dissector, and belongs to the technical field of medical instruments.

### Background of the invention

At present, in the field of medical endoscopy, an endoscopic submucosal dissection (ESD) is adopted to treat a lesion tissue in the digestive tract, by peeling, through a dissector, the mucous membranes where the lesion tissue is located, thereby achieving the purpose of treatment. The mucosa dissector is commonly used for removing tissues or polyps under affected mucous membranes, and is a clinically common instrument. Conventional mucosa dissectors are complex in operation, long in operation time, high in risk, and have relatively high technical requirements for clinicians, making it difficult to widely popularize and use the technology.

### Summary of the invention

The technical problem to be solved by the present invention is to overcome the defects in the prior art, and to provide a mucosa dissector. When endoscopic cutting and removing for the nidus of the submucosal tissues is implemented, the mucosa dissector clings to the tissue conveniently, is located accurately, make the operation more convenient and fast, greatly reducing the operation difficulty for the doctor. It saves time, and avoids the phenomenon of burning out other non-diseased tissues.

To solve the above technical problem, the solution of the present invention provides a mucosa dissector, comprising:
an outer sleeve assembly;
an electric loop, a part of the electric loop is provided with an insulating layer, and the rest of the electric loop is in exposed status for the dissecting the mucous membranes;
a sliding ring, which is provided on the outer sleeve assembly, and is movable axially relative to the outer sleeve assembly, and an electrode plug is provided on the sliding ring;
a pulling cable, which is movable axially to be inserted into the outer sleeve assembly, one end of the pulling cable is connected with the electric loop, and the other end of the pulling cable is electrically connected with the electrode plug on the sliding ring.

Furthermore, the outer sleeve assembly comprises a tube sheath, a protective sleeve, a rotating cap and a core rod, and the tube sheath is connected with one end of the protective sleeve, and the other end of the protective sleeve is connected with the core rod through the rotating cap.

In addition, the electric loop is round, rhombic, half-moon, pentagonal or hexagonal.

With the above technical solution, the electric loop can extend out of or withdraw from the front end of the tube sheath under the manipulation of the sliding ring, the electric loop is provided with the insulating layer for the purpose of insulation in use; while the electric loop is turned on, the mucous membranes are smoothly removed by the exposed part of the electric loop. When cutting the submucosal tissue, it clings conveniently to the tissue and is accurately located. In this manner, it makes the operation more convenient and fast, greatly reduces the operation difficulty for the doctor, saves time, and avoids the phenomenon of burning out other non-diseased tissues.

### BRIEF DESCRIPTION OF THE DRAWINGS

Fig. 1 is a structural schematic view of a first structure of the mucosa dissector of the present invention;
Fig. 2 is an enlarged view of the portion A in Fig. 1;
Fig. 3 is a structural schematic view of a second structure of the mucosa dissector of the present invention;
Fig 4 is an enlarged view of the portion B in Fig. 3;
Fig. 5 is a structural schematic view of a third structure of the mucosa dissector of the present invention;
Fig. 6 is an enlarged view of the portion C in Fig. 5;

### DETAILED DESCRIPTION

In order to make the contents of the present invention more readily apparent, the present invention is described in further details with reference to specific embodiments taken in conjunction with the drawings.

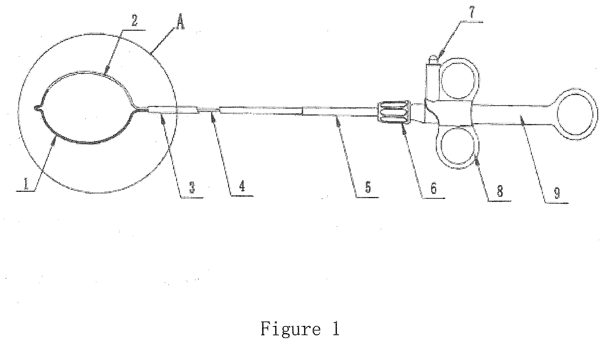

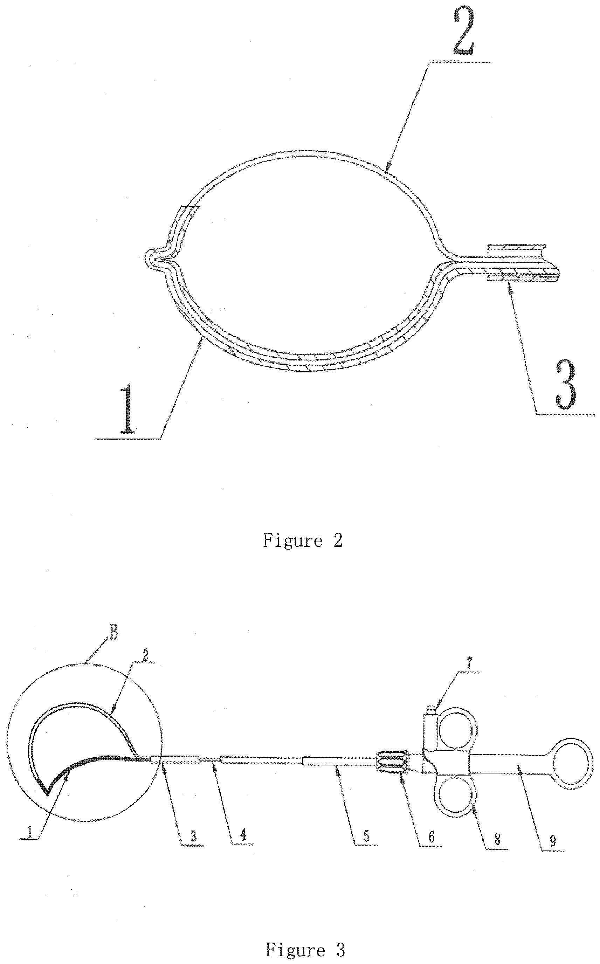

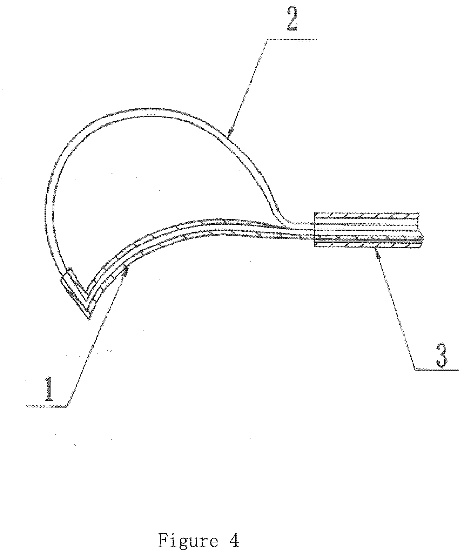

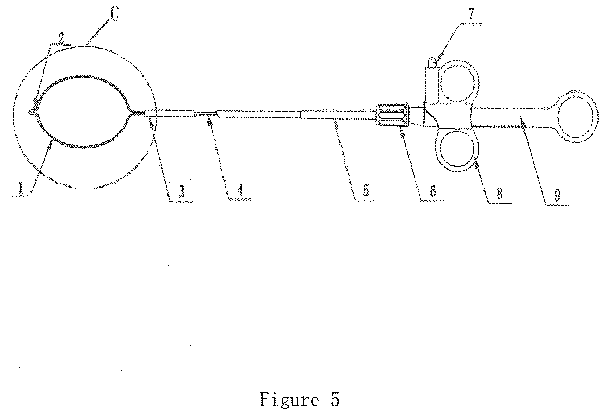

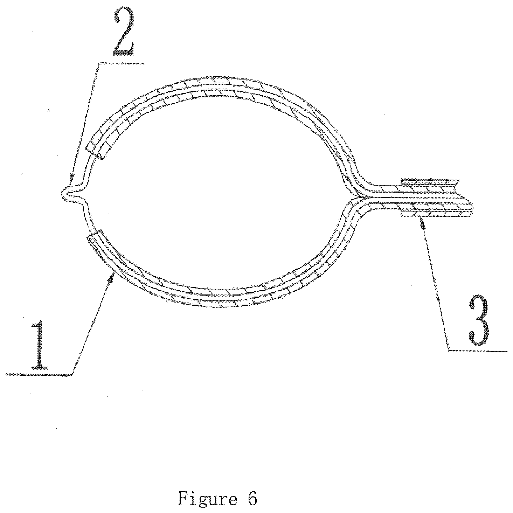

As shown in figures 1-6, the mucosa dissector comprises:
an outer sleeve assembly;
an electrical loop 2, and a portion of which is provided an insulating layer 1, and the rest of the electrical loop 2 is in an exposed status so as to dissect and peel the mucosa; there are certainly many types of arranging positions of the insulating layer 1 on the electric loop 2, such as one type as shown in Fig. 1 and Fig. 2 and another type as shown in Fig. 3 and Fig. 4, the specific structures of the two are: an upper portion of the electric loop 2 is mostly in an exposed state, and the remaining portion of the electric loop 2 except for the exposed portion has an insulating layer 1 thereon; the specific structure of the another type as shown in Fig. 5 and Fig. 6 is: most of the upper and lower portions of the electric loop 2 are provided with an insulating layer 1, and only the partial portion connected to both the front ends of the upper and lower portions is in an exposed state; the insulating layer 1 may be provided at any position on the electric loop 2;
a sliding ring 8, which is provided on the outer sleeve assembly and is movable axially relative to the outer sleeve assembly, with an electrode plug 7 provided on the sliding ring 8;
a pulling cable 4, which is movable axially to be inserted into the outer sleeve assembly, one end of the pulling cable 4 is connected with the electric loop 2, and the other end of the pulling cable 4 is electrically connected with the electrode plug 7 on the sliding ring 8.

As shown in Fig. 1, the outer sleeve assembly comprises a tube sheath 3, a protective sleeve 5, a rotating cap 6 and a core rod 9, and the tube sheath 3 is connected with one end of the protective sleeve 5, and the other end of the protective sleeve 5 is connected with the core rod 9 through the rotating cap 6.

The electric loop 2 may be but not limited to be round, rhombic, half-moon, pentagonal or hexagonal.

The working principle of the present invention is as follows:
the electric loop 2 can slowly extend out of the front end of the tube sheath 3 under the manipulation of the sliding ring 8, a high-frequency power supply is switched on and connected through the electrode plug 7 while the electric loop is pushed out, an exposed portion of the electric ring 2 is used to cut and dissect electrically the submucosal tissues. By manipulating the extension length of the electric loop 2 and adjusting the position of the electric loop 2, the submucosal tissues are dissected and peeled gradually. The electric loop 2 is provided with the insulating layer 1, which functions to maintain insulation during use for the purpose of protection. In this manner, during the cutting of the submucosal tissues or polypus, the mucosa dissector clings to the tissue conveniently and is accurately positioned, thereby making the operation more convenient and fast, greatly reducing the operation difficulty for the doctor, saving time, and avoiding the phenomenon of burning out other non-diseased tissues.

The above specific embodiments further provide detailed description with regard to the technical problem to be solved by the present invention, technical solution and beneficial effects thereof, it is to be understood that the foregoing description is merely of the specific embodiments of the present invention and is not intended to be limit the present invention, and that any modifications, equivalent substitutions, improvements and the like within the spirit and principles of the present invention shall be included within the protection scope of the present invention.

## Claims

1. A mucosa dissector, wherein, it comprises:
an outer sleeve assembly;
an electrical loop (2), a portion of which is provided with an insulating layer (1) and the rest of which is in an exposed status so as to dissect the mucosa;
a sliding ring (8), which is provided on the outer sleeve assembly, the sliding ring (8) is movable axially relative to the outer sleeve assembly, and an electrode plug (7) is provided on the sliding ring (8); and
a pulling cable (4), which is movable axially to be inserted into the outer sleeve assembly, one end of the pulling cable (4) is connected with the electric loop (2), and the other end of the pulling cable (4) is electrically connected with the electrode plug (7) on the sliding ring (8).

2. The mucosa dissector of claim 1, wherein the outer sleeve assembly comprises a tube sheath (3), a protective sleeve (5), a rotating cap (6) and a core rod (9), and the tube sheath (3) is connected with one end of the protective sleeve (5), and the other end of the protective sleeve (5) is connected with the core rod (9) through the rotating cap (6).

3. The mucosa dissector of claim 1 or claim 2, wherein the electric loop (2) is round, rhombic, half-moon, pentagonal or hexagonal.
